# EUROPÄISCHE PATENTSCHRIFT

(11) **EP 3 155 401 B1**
(45) Veröffentlichungstag und Bekanntmachung des Hinweises auf die Patenterteilung: **30.10.2019**
(21) Anmeldenummer: 15730744.8
(22) Anmeldetag: 16.06.2015
(51) Int. Cl.: G01N 21/17, G01N 21/552, G01N 21/63, G01N 33/49, A61B 5/1455, A61B 5/145, G01N 33/18, G01N 33/28

(54) **NICHT-INVASIVE STOFFANALYSE**
NON-INVASIVE SUBSTANCE ANALYSIS
ANALYSE NON INVASIVE D'UNE SUBSTANCE

(30) Priorität: 16.06.2014 DE 102014108424
(43) Veröffentlichungstag der Anmeldung: 19.04.2017
(73) Patentinhaber: Diamontech GmbH, 10245 Berlin (DE)
(72) Erfinder: MÄNTELE, Werner, 83088 Kiefersfelden-Mühlbach (DE); PLEITEZ RAFAEL, Miguel Angel, 81735 München (DE); LIEBLEIN, Tobias, 60487 Frankfurt am Main (DE); HERTZBERG, Otto, 60318 Frankfurt (DE); BAUER, Alexander, 61440 Oberursel (DE); VON LILIENFELD-TOAL, Hermann, 63571 Gelnhausen (DE); KÜDERLE, Arne, 65934 Frankfurt am Main (DE); PFUHL, Tabea, 64807 Dieburg (DE)
(74) Vertreter: Lucke, Andreas
(86) Internationale Anmeldenummer: PCT/EP2015/063470
(87) Internationale Veröffentlichungsnummer: WO 2015/193310

(56) Entgegenhaltungen:
- DE-A1- 3 146 700
- FARAHI R H ET AL: "Paper;Pump probe photothermal spectroscopy using quantum cascade lasers;Pump probe photothermal spectroscopy using quantum cascade lasers", JOURNAL OF PHYSICS D: APPLIED PHYSICS, INSTITUTE OF PHYSICS PUBLISHING LTD, GB, Bd. 45, Nr. 12, 6. März 2012 (2012-03-06), Seite 125101, XP020219064, ISSN: 0022-3727, DOI: 10.1088/0022-3727/45/12/125101
- X. GUO ET AL: "Non-invasive Glucose Measurements Using Wavelength Modulated Differential Photothermal Radiometry (WM-DPTR)", INTERNATIONAL JOURNAL OF THERMOPHYSICS, 24. August 2012 (2012-08-24), XP055211861, ISSN: 0195-928X, DOI: 10.1007/s10765-012-1276-z
- KAWAZUMI H ET AL: "Development of an interfacial thermal lens technique: monitoring the dissolving process of amphiphilic molecules at the hexane-water interface", CHEMICAL PHYSICS LETTERS, ELSEVIER BV, NL, Bd. 282, Nr. 2, 9. Januar 1998 (1998-01-09), Seiten 159-163, XP027355089, ISSN: 0009-2614, DOI: 10.1016/S0009-2614(97)01239-6 [gefunden am 1998-01-09]
- N A GEORGE ET AL: "Fibre optic position sensitive detection of photothermal deflection", APPLIED PHYSICS B: LASERS AND OPTICS, Bd. 77, Nr. 1, 20. August 2003 (2003-08-20), Seiten 77-80, XP055211868, ISSN: 0946-2171, DOI: 10.1007/s00340-003-1117-7

## Beschreibung

### GEBIET DER ERFINDUNG

Die vorliegende Erfindung betrifft ein Verfahren und ein System zur nicht-invasiven Analyse eines Stoffes. Insbesondere betrifft sie ein Verfahren und ein System zur nicht-invasiven Analyse eines Stoffes aus der Ablenkung eines Mess-Lichtstrahls, der in einem an den Stoff angelegten optischen Medium reflektiert wird.

### HINTERGRUND

In verschiedensten technischen Anwendungen und insbesondere in chemischen, biologischen und medizinischen Anwendungen wird die Analyse von Stoffen hinsichtlich ihrer Zusammensetzung und Inhaltsstoffe benötigt. In manchen Analyseverfahren wird dazu ein Teil des Stoffes entnommen und mit anderen Stoffen zur Reaktion gebracht. Aus der Veränderung des Reaktionsgemischs kann dann auf die Inhaltsstoffe des Stoffes geschlossen werden.

Die Entnahme oder Veränderung des Stoffes kann aber unerwünscht sein, beispielsweise wenn der Stoff durch die Reaktion für seinen eigentlichen Zweck nicht mehr verwendbar ist oder wenn die Entnahme eines Teils des Stoffes den Stoff beschädigt oder zerstört. In diesen Fällen kann eine nicht-invasive Stoffanalyse vorteilhaft sein, bei der die ursprüngliche Funktion oder Verwendungsmöglichkeit des Stoffes durch die Analyse nicht beeinträchtigt wird.

Die DE 31 46 700 A1 offenbart ein Verfahren nach dem Oberbegriff des Anspruchs 1. Gemäß dieser Schrift wird eine Probe durch monochromatisches, mithilfe eines Choppers amplitudenmoduliertes Anregungslicht durch eine ATR-Platte hindurch beleuchtet. Das amplitudenmodulierte Anregungslicht wird in der Probe absorbiert und erzeugt dadurch an der Grenzfläche zwischen der Probe und der ATR-Platte kleine Änderungen im Brechungsindex mit der Periode der Modulationsfrequenz. Ein Mess-Lichtstrahl wird mit einem Winkel in der Nähe des kritischen Winkels für die Totalreflexion in die ATR-Platte eingekoppelt. Wenn sich die Brechzahl infolge thermooptischer Effekte ändert, ändern sich die Bedingungen für die Totalreflexion bzw. die Aufteilung zwischen einem totalreflektiertem und einem evaneszenten Wellenanteil, sodass ein Teil des Lichts des Mess-Lichtstrahls an der Grenzfläche zur Probe aus der ATR-Platte austritt und somit die an einem Detektor gemessene Intensität abnimmt.

George et al.: "Fibre optic position sensitive detection of photothermal deflection", Applied Physics B: Lasers and Optics, Bd.77, Nr. 1, 20. August 2003, Seiten 77-80, lehrt die thermische Charakterisierung von Festkörpern durch Benetzen mit einem Koppelfluid, Erzeugen von thermischen Wellen im Festkörper durch Absorption eines Anregungslichtstrahls, und Detektion einer Ablenkung eines parallel zur Festkörperoberfläche durch das Koppelfluid transmittierten Messlichtstrahls mithilfe einer optischen Faser.

### ZUSAMMENFASSUNG DER ERFINDUNG

Der vorliegenden Erfindung liegt somit die Aufgabe zugrunde, ein Verfahren und ein System zur nicht-invasiven Analyse eines Stoffes bereitzustellen.

Diese Aufgabe wird durch ein Verfahren gemäß Anspruch 1 und ein System gemäß Anspruch 9 gelöst. Bevorzugte Ausführungsformen sind in den abhängigen Ansprüchen angegeben.

Erfindungsgemäß umfasst das Verfahren einen Schritt des Anordnens einer Stoffoberfläche auf einem optischen Medium, so dass zumindest ein Bereich der Oberfläche des optischen Mediums in Kontakt mit der Stoffoberfläche steht. Unter dem Schritt des Anordnens einer Stoffoberfläche auf dem optischen Medium soll im Sinne der vorliegenden Erfindung insbesondere auch das Anordnen des optischen Mediums auf einer Stoffoberfläche fallen und allgemein jegliches in Kontakt bringen. So kann das optische Medium insbesondere auch als Aufnahme für einen Stoff ausgestaltet sein, oder mit einem Stoff fest verbunden sein. Vorzugsweise ist das optische Medium ein Körper aus ZnS, ZnSe, Ge oder Si, der in einem vorbestimmten Wellenlängenbereich, vorzugsweise im Infrarotbereich oder einem Teil des Infrarotbereichs, transparent ist. Der Stoff kann ein Gewebe sein, beispielsweise Haut, aber auch eine Flüssigkeit oder ein Festkörper oder eine Kombination aus Festkörper, Haut und Gewebe, wie beispielsweise ein Probenhalter aus Glas mit einem daran befindlichen zu untersuchenden Gewebe oder einer darin befindlichen Flüssigkeit.

Bei dem genannten Kontakt handelt es sich typischerweise um einen direkten Kontakt. Als "direkter Kontakt" wird ein solcher Kontakt angesehen bei dem sich die Oberflächen des optischen Mediums und des Stoffes berühren. In diesem Zusammenhang bedeutet der oben genannte Schritt des Anordnens des optischen Mediums auf der Stoffoberfläche das in Berührung bringen der Oberflächen des optischen Mediums und des Stoffes. Beispielsweise können das optische Medium und der Stoff jeweils in einem Bereich (im Wesentlichen) flache Oberflächen aufweisen, die miteinander in Kontakt gebracht werden.

Das erfindungsgemäße Verfahren umfasst ferner den Schritt des Aussendens eines Anregungs-Lichtstrahls mit einer Anregungswellenlänge durch den Bereich der Oberfläche des optischen Mediums, der in Kontakt mit der Stoffoberfläche steht, auf die Stoffoberfläche. Des Weiteren umfasst das erfindungsgemäße Verfahren den Schritt des Aussendens eines Mess-Lichtstrahls durch das optische Medium auf den Bereich der Oberfläche des optischen Mediums, der in Kontakt mit der Stoffoberfläche steht, derart dass sich Mess-Lichtstrahl und Anregungs-Lichtstrahl auf der Grenzfläche des optischen Mediums und der Stoffoberfläche überlappen. Vorzugsweise überlappen sich Mess-Lichtstrahl und Anregungs-Lichtstrahl an der Grenzfläche des optischen Mediums und der Stoffoberfläche zu 10% bis 100% oder 50% bis 100%, besonders vorzugsweise zu mehr als 90% oder sogar zu 100%. Der Grad der Überlappung wird beispielsweise dadurch ermittelt, dass ein erster Grenzflächenbereich bestimmt wird, in dem 95%, vorzugsweise 98% der gesamten Lichtintensität des Mess-Lichtstrahls auftrifft. Ebenso wird ein zweiter Grenzflächenbereich bestimmt, in dem zwischen 95%, vorzugsweise 98% der gesamten Lichtintensität des Anregungs-Lichtstrahls auftrifft. Die Schnittmenge des ersten und zweiten Grenzflächenbereichs wird dann ins Verhältnis zum Durchschnitt des ersten und zweiten Grenzflächenbereichs gesetzt und ergibt den Grad der Überlappung.

Das erfindungsgemäße Verfahren umfasst ferner den Schritt des direkten oder indirekten Detektierens einer Ablenkung des reflektierten Mess-Lichtstrahls in Abhängigkeit von der Wellenlänge des Anregungs-Lichtstrahls. Die Ablenkung kann beispielsweise direkt mittels eines positionsempfindlichen Photodetektors (PSD) oder indirekt mittels eines Photodetektors, insbesondere einer Photodiode ermittelt werden, der hinter einer Irisblende angeordnet ist.

Des Weiteren umfasst das erfindungsgemäße Verfahren den Schritt des Analysierens des Stoffes anhand der detektierten Ablenkung des reflektierten Mess-Lichtstrahls in Abhängigkeit von der Wellenlänge des Anregungs-Lichtstrahls. Analysieren bedeutet hier insbesondere das Messen oder Bestimmen von Parametern, die die Stoffzusammensetzung kennzeichnen. Vorzugsweise umfasst das Analysieren das Bestimmen einer Absorptionscharakteristik des Stoffes. Ist der Anregungs-Lichtstrahl ein Infrarot-Lichtstrahl, dann umfasst das Analysieren vorzugsweise das Durchführen einer Infrarotspektroskopie.

Das erfindungsgemäße Verfahren basiert darauf, dass ein in einem Stoff absorbierter Anregungs-Lichtstrahl den Strahlengang eines Mess-Lichtstrahls durch das optische Medium verändert. Ursächlich hierfür ist, dass die Absorption des Anregungs-Lichtstrahls im Stoff eine Temperaturerhöhung induziert, die die Brechzahl bzw. den Brechungsindex des mit dem Stoff in Kontakt stehenden optischen Mediums lokal verändert und somit den Strahlengang des Mess-Lichtstrahls ablenkt. Der Grad der Ablenkung korreliert mit dem Grad der Absorption des Anregungs-Lichtstrahls im Stoff, so dass Stoffbestandteile mit charakteristischem Absorptionsspektrum über den Grad der Ablenkung des Mess-Lichtstrahls identifiziert werden können.

Vorzugsweise umfasst das Verfahren den Schritt des Ausrichtens des Mess-Lichtstrahls, so dass der Mess-Lichtstrahl an der Grenzfläche zwischen dem optischen Medium und der Stoffoberfläche totalreflektiert wird. Der Begriff der Totalreflexion wird in der vorliegenden Beschreibung und den Ansprüchen in Anlehnung an das Snelliussche Brechungsgesetz mit einem Einfallswinkel des Mess-Lichtstrahls gleichgesetzt, der größer ist als der Grenzwinkel der Totalreflexion, welcher gleich dem Arkussinus des Quotienten aus dem Brechungsindex des Stoffes geteilt durch den Brechungsindex des optischen Mediums ist. Vorzugsweise ist der Mess-Lichtstrahl so ausgerichtet, dass er die Analyse einer Vielzahl unterschiedlicher Stoffe mit unterschiedlichen optischen Dichten ermöglicht.

Vorzugsweise ist der Anregungs-Lichtstrahl als intensitätsmodulierter, insbesondere gepulster Anregungs-Lichtstrahl ausgebildet. Vorzugsweise liegt die Modulationsfrequenz, insbesondere die Pulsrate, zwischen 5 und 2000 Hz und besonders vorzugsweise zwischen 10 und 1000 Hz oder 20 und 700 Hz. Ist der Anregungs-Lichtstrahl als gepulster Anregungs-Lichtstrahl ausgebildet, erzeugt die zyklische Erwärmung und Abkühlung der das Anregungslicht absorbierenden Stoffbestandteile durch die Ausdehnung und Schrumpfung der Stoffbestandteile Druck- und insbesondere Wärmewellen, die durch den Stoff laufen und sich in das optische Medium ausbreiten und somit den Strahlengang des Mess-Lichtstrahls ebenfalls ablenken.

Vorzugsweise wird der Schritt des Aussendens des Anregungs-Lichtstrahls für verschiedene Modulationsfrequenzen wiederholt und der Schritt des Analysierens des Stoffes umfasst das Analysieren des Stoffes anhand der detektierten Ablenkungen des Mess-Lichtstrahls in Abhängigkeit von der Wellenlänge und der Modulationsfrequenz des Anregungs-Lichtstrahls. Dabei führen verschiedene Modulationsfrequenzen zur Einbeziehung verschiedener Schichten in den Absorptionsprozess. So führen beispielsweise höhere Modulationsfrequenzen zu eher oberflächennahen Absorptionsprozessen, während niedrigere Modulationsfrequenzen auch Absorptionsprozesse in tieferen Schichten umfassen. Somit wird eine Analyse verschiedener Schichten ermöglicht.

Vorzugsweise umfasst der Schritt des Analysierens des Stoffes ein Subtrahieren eines Wertes, der auf einer Ablenkung des Mess-Lichtstrahls basiert, welche bei einer ersten Modulationsfrequenz detektiert wurde, von einem Wert, der auf einer Ablenkung des Mess-Lichtstrahls basiert, welche bei einer zweiten Modulationsfrequenz detektiert wurde, oder ein Dividieren eines Wertes, der auf einer Ablenkung des Mess-Lichtstrahls basiert, welche bei einer ersten Modulationsfrequenz detektiert wurde, durch einen Wert, der auf einer Ablenkung des Mess-Lichtstrahls basiert, welche bei einer zweiten Modulationsfrequenz detektiert wurde. Durch die Differenzbildung bzw. Division kann der Einfluss oberflächennaher Schichten aus Absorptionsprozessen, die in oberflächennahen und tieferen Schichten stattfinden, herausgerechnet werden, so dass sich der Beitrag der tieferen Schichten bestimmen lässt.

Vorzugsweise umfasst der Schritt des Analysierens des Stoffes ein Subtrahieren von Werten, die auf Ablenkungen des Mess-Lichtstrahls basieren, welche bei einer ersten Modulationsfrequenz für unterschiedliche Wellenlängen des Anregungs-Lichtstrahls detektiert wurden, von Werten, die auf Ablenkungen des Mess-Lichtstrahls basieren, welche bei einer zweiten Modulationsfrequenz für unterschiedliche Wellenlängen des Anregungs-Lichtstrahls detektiert wurden, wobei die Werte besonders vorzugsweise Absorptionsintensitätsspektralwerte sind, oder ein Dividieren von Werten, die auf Ablenkungen des Mess-Lichtstrahls basieren, welche bei einer ersten Modulationsfrequenz für unterschiedliche Wellenlängen des Anregungs-Lichtstrahls detektiert wurden, durch Werte, die auf Ablenkungen des Mess-Lichtstrahls basieren, welche bei einer zweiten Modulationsfrequenz für unterschiedliche Wellenlängen des Anregungs-Lichtstrahls detektiert wurden, wobei die Werte besonders vorzugsweise Absorptionsintensitätsspektralwerte sind. Durch das Subtrahieren bzw. Dividieren der Werte kann ein Absorptionsspektrum einer bestimmten Schicht des Stoffes isoliert werden, so dass das Auftreten bestimmter Substanzen in dieser Schicht nachgewiesen werden kann, was insbesondere dann vorteilhaft ist, wenn das Auftreten der Substanzen im Stoff nicht gleichmäßig verteilt ist.

Vorzugsweise umfasst der Schritt des Analysierens das Zuordnen von Werten, die auf Ablenkungen des Mess-Lichtstrahls basieren, welche bei verschiedenen Modulationsfrequenzen detektiert wurden, mit verschiedenen Bereichen im Stoff, vorzugsweise unterschiedlich tief liegenden Bereichen im Stoff. Durch das Zuordnen oder Assoziieren der Werte mit Stoffbereichen, kann eine Verteilung einer Substanz im Stoff bereitgestellt werden, insbesondere ein Tiefenprofil eines Vorhandenseins einer Substanz.

Vorzugsweise umfasst die Bestimmung der Ablenkung des Mess-Lichtstrahls das Verstärken eines zugehörigen Messsignals mit einem Lock-in-Verstärker. Durch die Verwendung eines gepulsten Anregungs-Lichtstrahls in Kombination mit einem Lock-in-Verstärker können auch kleine Signale bzw. Signaländerungen, die im Bereich des Rauschens liegen, erfassbar gemacht werden. Der gepulste Anregungs-Lichtstrahl wird vorzugsweise durch einen optischen Chopper moduliert. Der optische Chopper ist vorzugsweise mit dem Lock-in-Verstärker gekoppelt. Alternativ kann der gepulste Anregungs-Lichtstrahl durch eine gepulste Anregungs-Lichtquelle erzeugt werden.

Vorzugsweise wird der Anregungs-Lichtstrahl durch eine optische Einrichtung auf der Oberfläche des optischen Mediums fokussiert, wobei die optische Einrichtung insbesondere einen Parabolspiegel umfasst. Vorzugsweise wird die optische Einrichtung mit Hilfe eines Justage-Lasers, der sichtbares Licht aussendet, justiert. Der Laserstrahl des Justage-Lasers kann durch eine Spiegelanordnung so ausgerichtet werden, dass der Strahlengang des Justage-Laserstrahls mit dem Strahlengang des Anregungs-Lichtstrahls zumindest teilweise zusammenfällt.

Vorzugsweise wird die Wellenlänge des Anregungs-Lichtstrahls variiert, insbesondere indem die Wellenlänge zyklisch innerhalb eines vorbestimmten Wellenlängenbereichs durchgestimmt wird oder indem charakteristische Wellenlängen, insbesondere Absorptions-Wellenlängen einer vermuteten Substanz, gezielt eingestellt werden. Durch das Variieren des Wellenlängenbereichs des Anregungs-Lichtstrahls kann eine Spektralanalyse erfolgen, die es ermöglicht, Stoffbestandteile mit teilweise ähnlichen oder überlappenden Absorptionsspektren zu unterscheiden. Das Variieren des vorbestimmten Wellenlängenbereichs kann beispielsweise durch Verwendung einer durchstimmbaren Lichtquelle erfolgen.

Vorzugsweise ist der Anregungs-Lichtstrahl ein Anregungs-Laserstrahl. Durch die Verwendung eines Anregungs-Laserstrahls kann der Absorptionsbereich bzw. ein Absorptionsspektrum mit hoher Auflösung analysiert werden. Für den infraroten Spektralbereich kann als Anregungs-Laser vorzugsweise ein Quantenkaskadenlaser verwendet werden.

Vorzugsweise ist der Mess-Lichtstrahl ein Mess-Laserstrahl. Vorzugsweise liegt die Wellenlänge des Mess-Lichtstrahls im sichtbaren Wellenlängenbereich. Durch die Verwendung eines sichtbaren Mess-Laserstrahls kann die Ausrichtung des Mess-Laserstrahls auf den Bereich der Grenzfläche, durch den der Anregungs-Lichtstrahl tritt, vereinfacht werden.

Vorzugsweise liegt die Anregungs-Wellenlänge in einem Bereich von 6 µm bis 13 µm, besonders vorzugsweise von 8 µm bis 11 µm.

Vorzugsweise wird die Polarisation des Mess-Lichtstrahls so eingestellt, dass die Ablenkung des reflektierten Mess-Lichtstrahls maximal ist.

Vorzugsweise wird der Mess-Lichtstrahl vor der Detektion der Ablenkung mindestens ein weiteres Mal, vorzugsweise zwei bis fünf weitere Male an dem gleichen Ort auf der Grenzfläche des optischen Mediums totalreflektiert. Beispielsweise, indem der Mess-Lichtstrahl durch ein Spiegelsystem auf den Überlappungsbereich mit dem Anregungs-Lichtstrahl zurückreflektiert wird. Dadurch wird der Ablenkwinkel effektiv vergrößert, wodurch die Genauigkeit der Detektion der Ablenkung erhöht wird.

Ist der zu analysierende Stoff die Haut eines Patienten, umfasst das Verfahren vorzugsweise den Schritt des Vorbereitens der Hautoberfläche durch Anbringen und Abziehen eines Gewebestreifens zur Entfernung abgestorbener Hautzellen, wobei der Gewebestreifen ein auf der Hautoberfläche haftendes Material aufweist. Durch die Entfernung der abgestorbenen Hautzellen kann die Analysegenauigkeit verbessert werden, da störende Einflüsse durch abgestorbene Hautzellen vermieden werden.

Ist der zu analysierende Stoff die Haut eines Patienten, umfasst der Schritt des Analysierens des Stoffes vorzugsweise den Schritt des Bestimmens des Blutzuckerspiegels des Patienten. Besonders vorzugsweise umfasst der Schritt des Bestimmens des Blutzuckerspiegels den Schritt des Messens des Glukosegehalts der interstitiellen Flüssigkeit der Haut des Patienten.

Ist der zu analysierende Stoff die Haut eines Patienten, umfasst der Schritt des Analysierens des Stoffes vorzugsweise den Schritt des Bestimmens eines Wassergehalts der Haut des Patienten.

Ist der zu analysierende Stoff die Haut eines Patienten, umfasst der Schritt des Analysierens des Stoffes vorzugsweise den Schritt des Bestimmens einer Proteinzusammensetzung der Haut des Patienten und besonders vorzugsweise ein Bestimmen einer Proteinzusammensetzung der Haut des Patienten in verschiedenen Hautschichten. Dabei versteht es sich, dass unter dem Begriff "Hautschichten" Bereiche in der Haut verstanden werden sollen, die sich (im Wesentlichen) parallel zur Hautoberfläche erstrecken und über- bzw. untereinander angeordnet sind.

Vorzugsweise ist der Stoff eine fließende oder stehende Flüssigkeit oder Emulsion und das Analysieren des Stoffes umfasst ein Bestimmen eines Zucker-, Alkohol-, Fett- und/oder Proteingehalts der Flüssigkeit.

Vorzugsweise ist der Stoff ein Kraftstoff und das Analysieren des Stoffes umfasst ein Bestimmen eines Alkohol-, Rapsölmethylester-, Blei- oder Benzolgehalts des Kraftstoffs.

Vorzugsweise ist der Stoff Süß- oder Salzwasser und das Analysieren des Stoffes umfasst ein Bestimmen einer Verunreinigung des Wassers.

Vorzugsweise ist der Stoff eine Körperflüssigkeit.

Vorzugsweise wird der Wellenlänge des Anregungs-Lichtstrahls ein Absorptionsintensitätswert zugeordnet, basierend auf der detektierten Ablenkung des Mess-Lichtstrahls. Der Absorptionsintensitätswert kann mit einem Kalibrier-Absorptionsintensitätswert verglichen werden, welcher den Absorptionsintensitätswert der Haut des Patienten bei bekanntem Blutzuckerspiegel und ebendieser Wellenlänge des Anregungs-Lichtstrahls repräsentiert. Vorzugsweise wird dann der aktuelle Blutzuckerspiegel des Patienten auf Basis des Vergleiches bestimmt, wobei der bestimmte Blutzuckerspiegel umso mehr von dem der Kalibrierung zugrundeliegenden Blutzuckerspiegel abweicht, je mehr der Absorptionsintensitätswert von dem Kalibrier-Absorptionsintensitätswert abweicht.

Das erfindungsgemäße System umfasst ein optisches Medium, eine Einrichtung zum Aussenden eines oder mehrerer Anregungs-Lichtstrahlen mit einer Anregungswellenlänge und eine Messeinrichtung. Wie bereits oben erörtert, kann das optische Medium beispielsweise ein Körper aus ZnS, ZnSe, Ge oder Si sein, der in einem vorbestimmten Wellenlängenbereich transparent ist.

Die Einrichtung zum Aussenden des Anregungs-Lichtstrahls ist so angeordnet, dass der ausgesendete Anregungs-Lichtstrahl durch eine erste Oberfläche in das optische Medium eindringt und dieses durch einen vorbestimmten Punkt auf einer zweiten Oberfläche wieder verlässt. Ist im Betrieb ein Stoff an der zweiten Oberfläche des optischen Mediums angeordnet, wird der Anregungs-Lichtstrahl auf der Oberfläche des Stoffs oder in dem Stoff zumindest teilweise absorbiert. Der Grad der Absorption kann mit einer Messeinrichtung detektiert werden.

Die Messeinrichtung umfasst eine Einrichtung zum Aussenden eines Mess-Lichtstrahls, welche so angeordnet ist, dass ein ausgesendeter Mess-Lichtstrahl in das optische Medium eindringt und im Betrieb mit dem Anregungs-Lichtstrahl auf der Grenzfläche des optischen Mediums und der Stoffoberfläche überlappt. Vorzugsweise überlappen sich Mess-Lichtstrahl und Anregungs-Lichtstrahl an der Grenzfläche des optischen Mediums und der Stoffoberfläche zu 10% bis 100% oder 50% bis 100%, besonders vorzugsweise zu mehr als 90% oder sogar zu 100%. Wie oben beschrieben wird der Grad der Überlappung beispielsweise dadurch ermittelt, dass ein erster Grenzflächenbereich bestimmt wird, in dem 95%, vorzugsweise 98% der gesamten Lichtintensität des Mess-Lichtstrahls auftrifft. Ebenso wird ein zweiter Grenzflächenbereich bestimmt, in dem zwischen 95%, vorzugsweise 98% der gesamten Lichtintensität des Anregungs-Lichtstrahls auftrifft. Die Schnittmenge des ersten und zweiten Grenzflächenbereichs wird dann ins Verhältnis zum Durchschnitt des ersten und zweiten Grenzflächenbereichs gesetzt und ergibt den Grad der Überlappung.

Beispielsweise kann der Mess-Lichtstrahl in einem Winkel, der kleiner ist als der Grenzwinkel der Totalreflexion, auf die Grenzfläche zwischen zweiter Oberfläche und Stoffoberfläche treffen, wobei der Auftreffort mit dem Auftreffort des Anregungs-Lichtstrahls überlappt und vorzugsweise mit ihm zusammenfällt.

Die Messeinrichtung umfasst ferner eine Einrichtung zum Empfangen des reflektierten Mess-Lichtstrahls und zum direkten oder indirekten Detektieren einer Ablenkung des reflektierten Mess-Lichtstrahls.

Wird beispielsweise das optische Medium mit der zweiten Oberfläche auf einem zu analysierenden Stoff angeordnet, so dringt der Anregungs-Lichtstrahl in den Stoff ein und wird je nach Zusammensetzung des Stoffs und Wellenlänge des Anregungs-Lichtstrahls unterschiedlich stark absorbiert. Die Absorption des Anregungs-Lichtes löst Wärmetransport und Druckwellen aus, welche den Strahlengang des Mess-Laserstrahls in dem optischen Medium beeinflussen. Da der Einfluss mit der Konzentration eines das Infrarot-Licht absorbierenden Stoffbestandteils korreliert, kann die Konzentration des Stoffbestandteils bestimmt werden, indem der Grad der Abweichung des Strahlenganges von einem unbeeinflussten Strahlengang gemessen wird.

Vorzugsweise wird der Mess-Lichtstrahl im Betrieb an der Grenzfläche zwischen dem optischen Medium und der Stoffoberfläche totalreflektiert.

Vorzugsweise ist der Anregungs-Lichtstrahl ein Infrarot-Lichtstrahl, da Infrarot-Licht von vielen Stoffen charakteristisch absorbiert wird und sich somit besonders zur Stoffanalyse eignet.

Vorzugsweise ist der Anregungs-Lichtstrahl ein intensitätsmodulierter, insbesondere gepulster Anregungs-Lichtstrahl. Vorzugsweise umfasst die Einrichtung zum Empfangen des reflektierten Mess-Lichtstrahls und zum direkten oder indirekten Detektieren der Ablenkung des reflektierten Mess-Lichtstrahls einen Lock-in-Verstärker. Vorzugsweise liegt die Modulationsfrequenz, insbesondere die Pulsrate, zwischen 5 und 2000 Hz, besonders vorzugsweise zwischen 10 und 1000 Hz oder 20 und 700 Hz. Zur Erzeugung eines gepulsten Anregungs-Lichtstrahls umfasst die Einrichtung zum Aussenden des Anregungs-Lichtstrahls vorzugsweise einen optischen Chopper. Vorzugsweise ist der optische Chopper im Strahlengang des Anregungs-Lichtstrahls platziert und moduliert die Intensität des Anregungs-Lichtstrahls. Durch die Verwendung eines intensitätsmodulierten und insbesondere eines gepulsten Lichtstrahls in Kombination mit einem Lock-in-Verstärker können auch kleine Signale bzw. Signaländerungen, die im Bereich des Rauschens liegen, erfassbar gemacht werden.

Vorzugsweise ist der Anregungs-Lichtstrahl ein Anregungs-Laserstrahl und ist die Einrichtung zum Aussenden des Anregungs-Laserstrahls dazu eingerichtet, Anregungs-Laserstrahlen unterschiedlicher Anregungs-Frequenzen auszusenden. Durch die Verwendung eines Anregungs-Laserstrahls kann der Absorptionsbereich bzw. ein Absorptionsspektrum mit hoher Auflösung analysiert werden.

Vorzugsweise umfasst das System ferner eine optische Einrichtung, die dazu bestimmt ist, den Anregungs-Lichtstrahl auf den vorbestimmten Punkt zu fokussieren. Durch die Fokussierung auf den vorbestimmten Punkt kann die Wirkung des Anregungs-Lichtstrahls noch stärker konzentriert werden, wodurch die Ablenkung des Mess-Lichtstrahls noch stärker ausfällt. Die optische Einrichtung kann beispielsweise einen Parabolspiegel umfassen.

Vorzugsweise umfasst das System einen Justage-Laser, welcher die Ausrichtung der optischen Einrichtung erleichtert. Dabei umfasst das System vorzugsweise ein Spiegelpaar, welches geeignet ist, den Strahlengang des Justage-Lasers so auszurichten, dass zumindest ein Teil des Strahlengangs des Justage-Lasers mit dem Strahlengang des Anregungs-Lichtstrahls zusammenfällt.

Vorzugsweise ist die Einrichtung zum Aussenden des Anregungs-Lichtstrahls ein Quantenkaskadenlaser. Vorzugsweise ist die Einrichtung zum Aussenden des Anregungs-Lichtstrahls in einem Anregungs-Wellenlängenbereich von 6 µm bis 13 µm, vorzugsweise 8 µm bis 11 µm durchstimmbar.

Vorzugsweise liegt die Wellenlänge des Mess-Lichtstrahls im sichtbaren Bereich. Dadurch wird die Ausrichtung des Mess-Lichtstrahls auf den Anregungs-Lichtstrahl vereinfacht.

Vorzugsweise umfasst die Einrichtung zum Empfangen des reflektierten Mess-Lichtstrahls und zum direkten oder indirekten Detektieren der Ablenkung des reflektierten Mess-Lichtstrahls einen Photodetektor, insbesondere eine Photodiode, und eine Irisblende, wobei der Photodetektor hinter der Irisblende angeordnet ist, oder ein PSD.

Vorzugsweise wird der Mess-Lichtstrahl vor der Detektion der Ablenkung mindestens ein weiteres Mal, vorzugsweise zwei bis fünf weitere Male an dem gleichen Ort auf der Grenzfläche des optischen Mediums totalreflektiert. Beispielsweise indem der Mess-Lichtstrahl durch ein Spiegelsystem unter einem anderen Winkel auf den Überlappungsort mit dem Anregungs-Lichtstrahl zurückreflektiert wird. Durch die mehrmalige Reflexion im Überlappungsbereich wird der Ablenkwinkel effektiv vergrößert, wodurch die Genauigkeit der Detektion der Ablenkung erhöht wird.

Ferner sind das Verfahren und das System zum Analysieren von Stoffen auf und in der Haut sowie zur Aufnahme von tiefenselektiven Profilen dieser Stoffe eingerichtet. Verfahren und System eignen sich ebenso zur Analyse von Spuren von Stoffen, wie Schad- oder Sprengstoffen auf der Haut. Oder zur Untersuchung der Aufnahme von Kosmetika, wie Fette und Inhaltsstoffe von Cremes, Salben oder Lotionen, oder pharmazeutischen Wirkstoffen, Medikamenten u. ä. in die Haut. Ferner eignen sich Verfahren und System ebenso für sensorische Anwendungen wie das Überwachen von fließenden und stehenden Flüssigkeiten bzw. Lösungen und Emulsionen zur Bestimmung z. B. des Alkoholgehalts oder der Zusammensetzung von alkoholischen Getränken wie beispielsweise Bier, Wein oder Branntwein, des Fettgehalts von Milch oder Milchprodukten, und allgemein des Zucker-, Fett-, Alkohol- oder Proteingehalts von Nahrungsmitteln. Des Weiteren eignen sich Verfahren und System für die Kraftstoffanalyse, beispielsweise für die Bestimmung des Blei- oder Benzolgehalts, das Messen von Wasserverunreinigungen (z.B. Öl) oder die Analyse von Körperflüssigkeiten sowie die Analyse von pathologischen und nicht-pathologischen Veränderungen der Haut, wie beispielsweise der Detektion von Melanomen mittels Bestimmung der Proteinzusammensetzung der Haut in verschiedenen Hauttiefen, der Detektion von Schuppenflechten oder Allergien sowie der Bestimmung der Hautfeuchtigkeit.

Vorzugsweise ist das System in einem Gerät zur Bestimmung des Blutzuckerspiegels eines Patienten umfasst, welches ferner eine Steuerung zum Einstellen unterschiedlicher Wellenlängen des Anregungs-Lichtstrahls und eine Logik- oder Recheneinheit umfasst, die dazu eingerichtet ist, aus detektierten Ablenkungen des Mess-Lichtstrahls in Abhängigkeit von der Anregungswellenlänge den Blutzuckerspiegel in der Haut eines Patienten zu bestimmen, wenn das optische Medium mit der Haut des Patienten derart in Kontakt gebracht wird, dass der an dem genannten vorbestimmten Punkt aus dem optischen Medium austretende Anregungs-Lichtstrahl in die Haut eindringt.

Vorzugsweise ist das System in einem Gerät zur Analyse eines Stoffes umfasst, das ferner eine Steuerung zum Einstellen unterschiedlicher Modulationsfrequenzen des Anregungs-Lichtstrahls und eine Logik- oder Recheneinheit umfasst, die dazu eingerichtet ist, den Stoff mittels detektierter Ablenkungen des Mess-Lichtstrahls bei unterschiedlichen Modulationsfrequenzen zu analysieren, wenn das optische Medium mit dem Stoff derart in Kontakt gebracht wird, dass der an dem genannten vorbestimmten Punkt aus dem optischen Medium austretende Anregungs-Lichtstrahl in den Stoff eindringt.

Vorzugsweise ist der Stoff die Haut eines Patienten und die Logik- oder Recheneinheit ist dazu eingerichtet, aus detektierten Ablenkungen des Mess-Lichtstrahls bei unterschiedlichen Modulationsfrequenzen, unterschiedliche Schichten der Haut des Patienten zu analysieren.

Vorzugsweise ist das System in einem Gerät zur Analyse von Inhaltsstoffen einer Flüssigkeit oder Emulsion umfasst, das ferner eine Steuerung zum Einstellen unterschiedlicher Wellenlängen des Anregungs-Lichtstrahls und eine Logik- oder Recheneinheit umfasst, die dazu eingerichtet ist, aus detektierten Ablenkungen des Mess-Lichtstrahls in Abhängigkeit von der Anregungswellenlänge Inhaltsstoffe der Flüssigkeit oder Emulsion zu bestimmen, wenn das optische Medium mit der Flüssigkeit oder Emulsion derart in Kontakt gebracht wird, dass der an dem genannten vorbestimmten Punkt aus dem optischen Medium austretende Anregungs-Lichtstrahl in die Flüssigkeit oder Emulsion eindringt.

### KURZBESCHREIBUNG DER FIGUREN

- Fig. 1: zeigt eine schematische Darstellung eines bevorzugten Ausführungsbeispiels des erfindungsgemäßen Systems im Betrieb;
- Fig. 2: zeigt Glukosebanden, die beim Analysieren menschlicher Haut mit dem bevorzugten Ausführungsbeispiel des erfindungsgemäßen Systems gemessen wurden;
- Fig. 3: zeigt einen Vergleich der nicht-invasiven Bestimmung des Blutglukosespiegels gemäß dem bevorzugten Ausführungsbeispiel des erfindungsgemäßen Systems und einer invasiven Messung mit einem Glukometer;
- Fig. 4: zeigt die Bewertung der nicht-invasiven Messung des Glukosespiegels gemäß Fig. 3 in einem Clark-Fehler-Diagramm;
- Fig. 5: zeigt Absorptionsintensitätsspektren, welche mit dem bevorzugten Ausführungsbeispiels des erfindungsgemäßen Systems bei verschiedenen Modulationsfrequenzen erhalten wurden
- Fig. 6a-c: zeigen eine schematische Darstellung eines zu analysierenden Stoffes, das Absorptionsintensitätsspektrum von Glukose, und das Absorptionsintensitätsspektrum einer Polymerschicht;
- Fig. 7: zeigt Prozessschritte des erfindungsgemäßen Verfahrens.

### BESCHREIBUNG BEVORZUGTER AUSFÜHRUNGSBEISPIELE

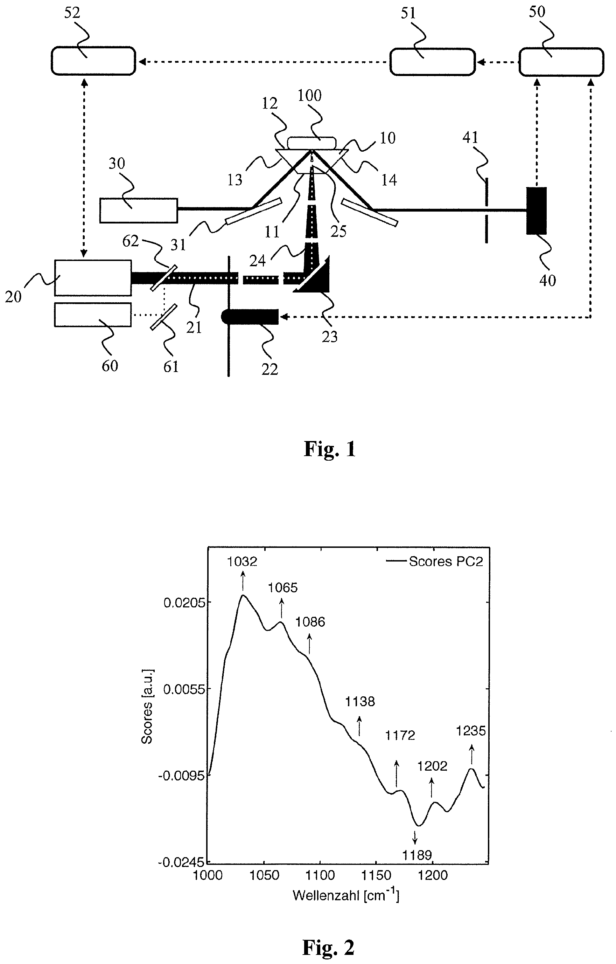

Fig. 1 zeigt eine schematische Darstellung eines bevorzugten Ausführungsbeispiels des erfindungsgemäßen Systems im Betrieb. Das bevorzugte Ausführungsbeispiel umfasst ein optisches Medium 10, eine Einrichtung zum Aussenden eines Infrarot-Lichtstrahls in Form eines Quantenkaskadenlasers 20, eine Einrichtung zum Aussenden eines Mess-Lichtstrahls in Form eines Messlasers 30 und eine Einrichtung mit Mitteln zum Empfangen des reflektierten Mess-Laserstrahls mit einer Photodiode 40 und mit Mitteln zum Auswerten des reflektierten Mess-Laserstrahls, die einen Lock-in-Verstärker 50, einen Digital-zu-Anlog-Wandler 51 und einen Rechner 52 umfassen.

Der Quantenkaskadenlaser 20 sendet einen Infrarot-Laserstrahl auf einem ersten Infrarot-Laserstrahl-Teilweg 21 durch einen optischen Chopper 22, welcher den kontinuierlichen Infrarot-Laserstrahl in einen gepulsten Infrarot-Laserstrahl umwandelt, vorzugsweise mit einer Pulsfrequenz zwischen 10Hz bis 1000Hz. Alternativ kann die Einrichtung zum Aussenden des Infrarot-Lichtstrahls, hier der Quantenkaskadenlaser 30, im Pulsbetrieb - ebenfalls mit einer Pulsfrequenz von vorzugsweise 10Hz bis 1000Hz - betrieben werden.

Am Ende des ersten Infrarot-Laserstrahl-Teilweges 21 trifft der Infrarot-Laserstrahl auf einen Parabolspiegel 23. Der Parabolspiegel 23 lenkt den Infrarot-Laserstrahl entlang eines zweiten Infrarot-Laserstrahl-Teilweges 24 auf eine erste Oberfläche 11 des optischen Mediums 10. Der Einfallswinkel zwischen dem zweitem Infrarot-Laserstrahl-Teilweg 24 und der ersten Oberfläche 11 beträgt in dem in Fig. 1 gezeigten bevorzugten Ausführungsbeispiel 90°. Grundsätzlich sind aber auch andere Einfallswinkel zwischen dem zweiten Infrarot-Laserstrahl-Teilweg 24 und der ersten Oberfläche 11 möglich, solange der Infrarot-Laserstrahl durch die erste Oberfläche 11 in das optische Medium 10 eindringt und nicht totalreflektiert wird.

Der Strahlengang des Infrarot-Laserstrahls verläuft in dem optischen Medium 10 entlang eines dritten Infrarot-Laserstrahl-Teilweges 25 auf eine zweite Oberfläche 12 des optischen Mediums 10 zu. Der Einfallswinkel zwischen dem dritten Infrarot-Laserstrahl-Teilweg 25 und der zweiten Oberfläche 12 beträgt in der in Fig. 1 gezeigten bevorzugten Ausführungsform 90°. Grundsätzlich sind aber auch hier andere Einfallswinkel zwischen dem dritten Infrarot-Laserstrahl-Teilweg 25 und der zweiten Oberfläche 12 möglich, solange ein ausreichender Anteil des Infrarot-Laserstrahls das optische Medium 10 durch die zweite Oberfläche 12 verlassen kann.

In der in Fig. 1 im Betrieb gezeigten bevorzugten Ausführungsform dringt der Infrarot-Laserstrahl entlang eines vierten Infrarot-Laserstrahl-Teilweges in eine Stoffprobe 100 ein, die an dem optischen Medium 10 anliegt. Der Abstand zwischen dem Parabolspiegel 23 und der zweiten Oberfläche 12 des optischen Mediums und die Form des Parabolspiegels 23 sind unter Berücksichtigung der optischen Dichten entlang des zweiten und dritten Infrarot-Laserstrahl-Teilweges 24, 25 so gewählt, dass der Infrarot-Laserstrahl auf die zweite Oberfläche 12 oder auf einen Punkt fokussiert ist, der in einem vorbestimmten Abstand, bspw. zwischen 30 µm und 100 µm, hinter der zweiten Oberfläche 12 in der Stoffprobe 100 liegt.

In der Stoffprobe wird der Infrarot-Laserstrahl von Stoffbestandteilen zumindest teilweise absorbiert. Die Absorption verändert die Temperatur des absorbierenden Stoffbestandteils. Bei einem gepulsten Infrarot-Laserstrahl, wie in Fig. 1 gezeigt, werden Druck- und Wärmewellen erzeugt, da sich die absorbierenden Stoffbestandteile zyklisch erwärmen und abkühlen und die dadurch entstehenden Ausdehnungsschwankungen zu Druckschwankungen führen, die sich in Form von Druckwellen durch den Stoff ausbreiten. Die erzeugten Wärme- und Druckwellen wandern, da das optische Medium 10 und der Stoff in direktem Kontakt stehen, in das optische Medium 10 und beeinflussen dort die Brechzahl.

Zur Messung der Absorption des Infrarot-Laserstrahls und die mit der Absorption korrelierte Veränderung der optischen Dichte des optischen Mediums 10, dient der von dem Messlaser 30 ausgesendete Mess-Laserstrahl. Dieser wird in Fig. 1 über einen ersten Spiegel 31 auf eine dritte Oberfläche 13 des optischen Mediums 10 gerichtet. Alternativ kann der Messlaser 30 auch so ausgerichtet sein, dass der Mess-Laserstrahl direkt auf die dritte Oberfläche 13 gerichtet ist. Wie in Fig. 1 gezeigt, kann der Strahlengang des Mess-Laserstrahls mit der dritten Oberfläche 13 einen Winkel von 90° einschließen. Alternativ kann der Strahlengang des Mess-Laserstrahls mit der dritten Oberfläche 13 einen kleineren Winkel einschließen, solange ein ausreichender Anteil des Mess-Laserstrahls in das optische Medium 10 eindringen kann.

Allerdings muss der Strahlengang des Mess-Laserstrahls so ausgerichtet sein, dass der Auftreffort des Mess-Laserstrahls auf der zweiten Oberfläche 12 des optischen Mediums 10 mit dem Auftreffort des Infrarot-Lichtstrahls auf der zweiten Oberfläche 12 des optischen Mediums zusammenfällt, oder ihn zumindest überlappt. Dadurch wird gewährleistet, dass während des Betriebs des Infrarot-Laserstrahls der Strahlengang des Mess-Laserstrahls durch den oben beschriebenen Bereich des optischen Mediums 10 führt, in dem die Brechzahl n durch die Absorption des Infrarot-Laserstrahls im Stoff 100 ausreichend stark beeinflusst wird, wobei klar ist, dass dieser Bereich begrenzt ist, da die Druckwellen bei ihrer Ausbreitung gedämpft werden und der Wärmeanstieg mit Abstand vom Absorptionsbereich abnimmt.

Vorzugsweise ist die optische Dichte des optischen Mediums 10 in Abhängigkeit vom zu untersuchenden Stoff 100 und dem Winkel zwischen dem Strahlengang des Mess-Laserstrahls und der zweiten Oberfläche 12 so gewählt, dass der Mess-Laserstrahl an der zweiten Oberfläche 12 bzw. an der Grenzfläche zwischen der zweiten Oberfläche 12 des optischen Mediums 10 und dem Stoff 100 totalreflektiert wird. Der reflektierte bzw. total reflektierte Mess-Laserstrahl trifft im weiteren Verlauf des Strahlengangs auf eine vierte Oberfläche 14 des optischen Mediums 10. Die vierte Oberfläche 14 kann ist so beschaffen, dass der Mess-Laserstrahl das optische Medium 10 durch die vierte Oberfläche 14 verlässt.

Wie in Fig. 1 gezeigt, wird der das optische Medium 10 verlassende Mess-Laserstrahl von der Photodiode 40 detektiert. Die Ablenkung des Mess-Laserstrahls durch die Veränderung der optischen Dichte in einem Teilbereich des optischen Mediums 10 kann, wie in Fig. 1 gezeigt, dadurch gemessen werden, dass der Mess-Laserstrahl vor Einfall auf die Photodiode 40 eine Irisblende 41 passiert. Der abgelenkte Mess-Laserstrahl wird dann teilweise durch die Irisblende 41 an einem Einfall in die Photodiode 40 gehindert, so dass die an der Photodiode 40 gemessene Intensität des Mess-Laserstrahls durch die Ablenkung beim Durchqueren des optischen Mediums 10 abnimmt. Alternativ kann eine ortsauflösende Photodiode 40, beispielsweise eine vier-Quadranten Photodiode dazu verwendet werden, die Änderung des Strahlenganges bzw. Ablenkung zu messen. In diesem Fall kann die Irisblende 41 entfallen.

Der Lock-in-Verstärker 50 empfängt das Signal der Photodiode 40 und das Frequenzsignal des gepulsten Infrarot-Lichtstrahls. Der Lock-in-Verstärker filtert das Rauschen aus dem Intensitätssignal, so dass auch kleine Intensitätsschwankungen messbar werden.

Das gefilterte Intensitätssignal der Photodiode 40 wird durch einen Digital-zu-AnalogWandler 51 in ein digitales Signal umgewandelt und an einen Rechner 52 übertragen, der dazu eingerichtet ist, die Intensitätsmesswerte in Abhängigkeit von der Wellenlänge bzw. dem Wellenlängenbereich des Quantenkaskadenlasers 30 aufzuzeichnen und die aufgezeichneten Werte mit einer Kalibrierkurve zu vergleichen, um daraus Rückschlüsse auf die Zusammensetzung des Stoffs 100 ziehen zu können. Der Rechner 52 umfasst dazu einen Prozessor, eine Speichereinheit und Instruktionen, welche, wenn sie von dem Prozessor ausgeführt werden, die Intensitätsmesswerte verknüpft mit der Wellenlänge bzw. dem Wellenlängenbereich des Quantenkaskadenlasers 30 aufzeichnen.

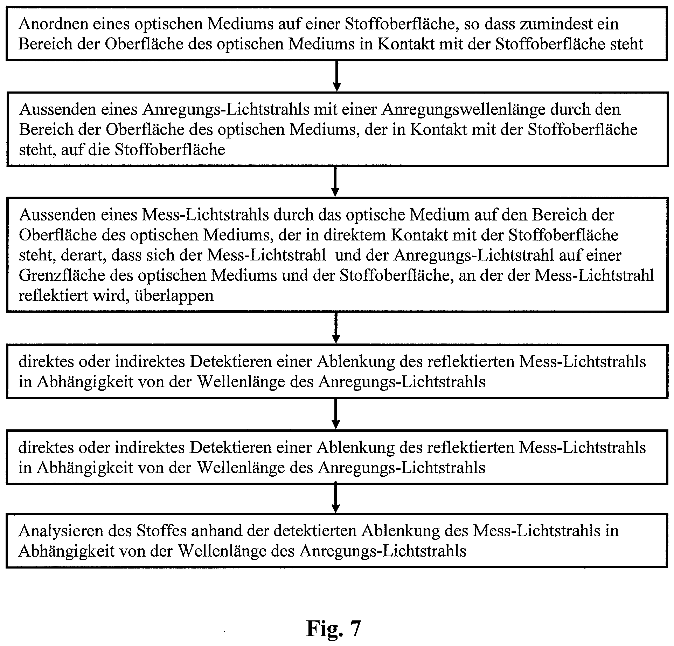

Der oben beschriebene Prozess, welcher in Fig. 7 übersichtsmäßig dargestellt ist, wird vorzugsweise für eine Reihe verschiedener Wellenlängen bzw. Wellenlängenbereiche im Infrarotlicht-Bereich wiederholt, um das Absorptionsspektrum einzelner oder mehrere Stoffbestandteile bestimmen zu können. Dabei kann das Vorhandensein eines Stoffbestandteiles durch das Messen charakteristischer Absorptionsspektren und die Konzentration des Stoffbestandteiles aus der Amplitude des Spektrums, beispielsweise durch Vergleich mit einer Kalibrierkurve, bestimmt werden. Der Quantenkaskadenlaser 30 kann dazu beispielsweise im Wellenlängenbereich von 8 µm bis 11 µm durchstimmbar sein.

Wenn das System zum Bestimmen eines Blutzuckerwertes eines Patienten verwendet wird, kann aus dem gemessenen Absorptionsspektrum der Glukose in der interstitiellen Flüssigkeit mittels einer Kalibrierkurve der Blutzuckerspiegel des Patienten berechnet werden.

Das in Fig. 1 gezeigte System enthält eine Einrichtung zum Aussenden eines Justier-Laserstrahls, hier in Form eines He-Ne-Lasers 60, und ein Spiegelpaar 61, 62, welches den Strahlengang des Justage-Lasers so ausrichtet, dass zumindest ein Teil des Strahlengangs des Justage-Lasers mit dem Strahlengang des Infrarot-Lichtstrahls, d.h. des Anregungs-Lichtstrahls zusammenfällt.

Ist der zu analysierende Stoff 100 Haut und das Ziel der Analyse die Bestimmung des Blutzuckerspiegels, wird beispielsweise der Daumenballen, die Fingerbeere, der Handballen oder eine andere Körperfläche mit der zweiten Oberfläche 12 des optischen Mediums 10 in Kontakt gebracht. Zuvor kann die in Kontakt zu bringende Körperfläche durch Anbringen und Abziehen eines Gewebestreifens zur Entfernung abgestorbener Hautzellen vorbereitet werden, wobei der Gewebestreifen ein auf der Hautoberfläche haftendes Material aufweist. Die Spektren der Haut, die auf diese Weise mit dem bevorzugten Ausführungsbeispiel des erfindungsgemäßen Systems gemessen wurden, zeigen als erste Hauptkomponente die Banden von Keratinozyten und Lipiden. Als zweite Hauptkomponente wurden die in Fig. 2 gezeigten Glukosebanden gemessen.

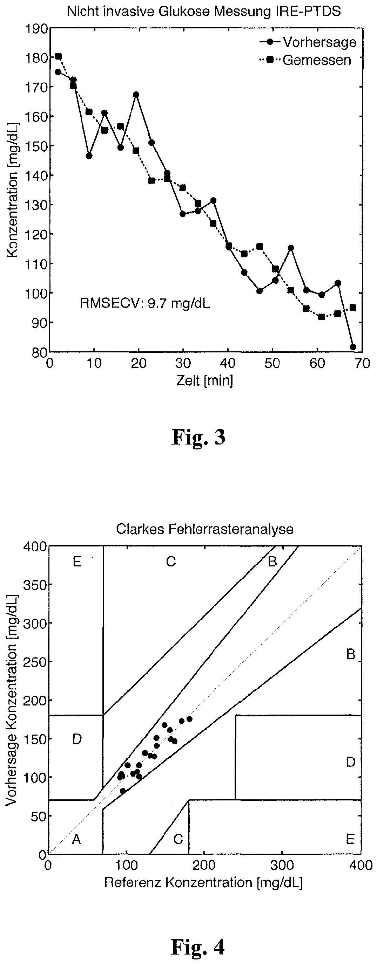

Bei einem Vergleich der nicht-invasiven Bestimmung des Blutglukosespiegels gemäß dem bevorzugten Ausführungsbeispiel des erfindungsgemäßen Systems und einer invasiven Messung mit einem Glukometer wurde, wie in Fig. 3 gezeigt, auch im Bereich geringer Konzentration (180 mg/dL bis unter 100 mg/dL) eine gute Übereinstimmung erzielt. Fig. 3 zeigt hierzu die gemessenen Blutzuckerwerte und die aus der Amplitude der zweiten Hauptkomponente bestimmte Blutzuckerkonzentration.

Trägt man, wie in Fig. 4 gezeigt, die Ergebnisse der nicht-invasiven Messung des Blutzuckerspiegels gemäß Fig. 3 in einem Clark-Fehler-Diagramm auf, liegen alle Datenpunkte der Messung in der "A"-Zone, in der bei einer akzeptierten Methode 90% der Werte liegen müssen.

Der Quantenkaskadenlaser 30 ist im Wellenlängenbereich von 8 µm bis 11 µm durchstimmbar. Aus dem gemessenen Absorptionsspektrum der Glukose in der interstitiellen Flüssigkeit wird mittels einer Kalibrierkurve der Blutzuckerspiegel des Patienten berechnet.

In einer bevorzugten Ausführungsform ist das System dauerhaft am Patienten angebracht, beispielsweise in Form eines Armbandes, und ferner dazu eingerichtet, den Patienten durch ein Warnsignal zu alarmieren, wenn die Glukosekonzentration in der interstitiellen Flüssigkeit unter einen vorbestimmten Wert fällt.

In einer weiteren bevorzugten Ausführungsform kann das System eine Insulinpumpe steuern, um einen gleichbleibenden Blutzuckerwert bei dem Patienten zu erhalten.

In einer weiteren bevorzugten Ausführungsform kann der Rechner 52 dazu eingerichtet sein, die Intensitätsmesswerte in Abhängigkeit von der Wellenlänge bzw. dem Wellenlängenbereich des Quantenkaskadenlasers 30 für verschiedene Pulsfrequenzen des Infrarot-Laserstrahls aufzuzeichnen. Der Rechner 52 umfasst dazu einen Prozessor, eine Speichereinheit und Instruktionen, welche, wenn sie von dem Prozessor ausgeführt werden, die Intensitätsmesswerte verknüpft mit der Wellenlänge bzw. dem Wellenlängenbereich und der Pulsfrequenz des Infrarot-Laserstrahls aufzeichnen.

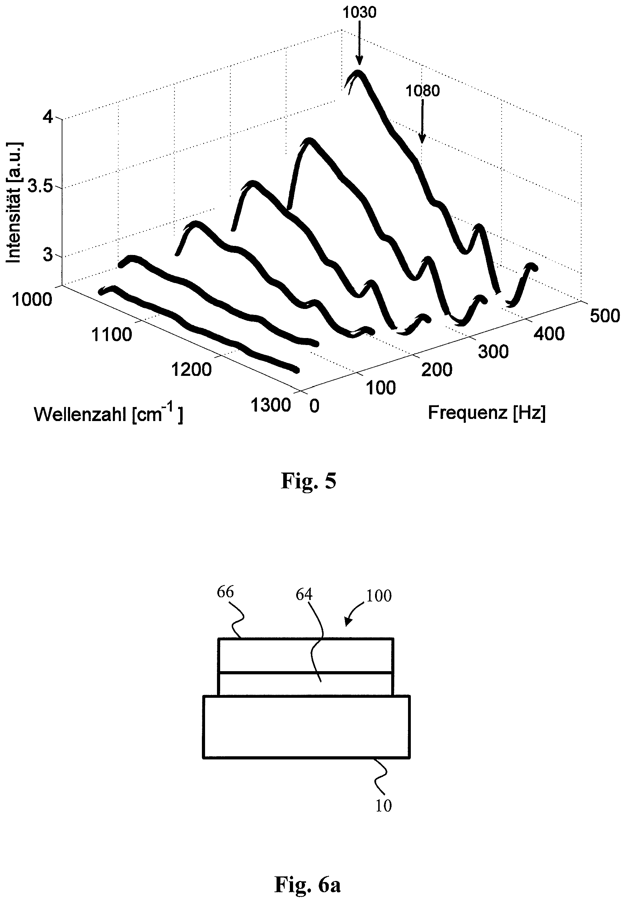

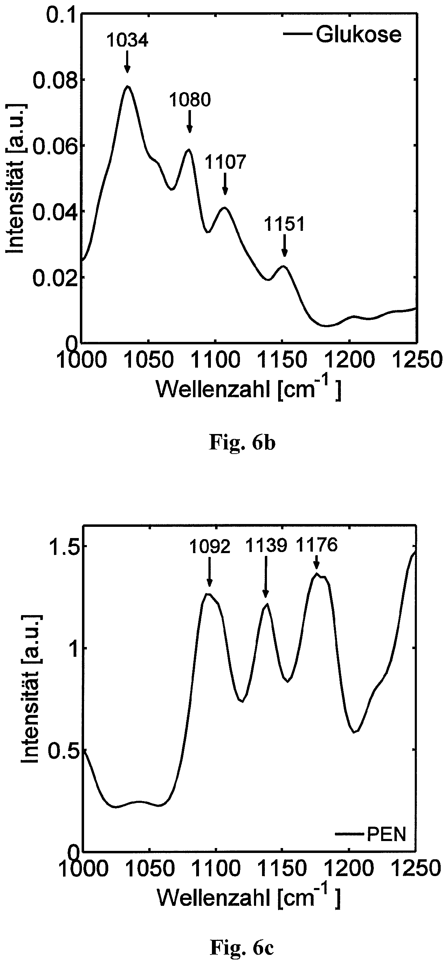

Fig. 5 zeigt dazu mit der weiteren bevorzugten Ausführungsform gemessene Intensitätsmesswerte der in Fig. 6a gezeigten Stoffanordnung, bestehend aus einer mit einer Polymerfolie 66 abgedeckten Glukoselösung 64, welche auf dem optischen Medium 10 angeordnet ist. Der Vergleich der in Fig. 5 gezeigten Absorptionsspektren mit den in Fig. 6b und 6c gezeigten Absorptionsintensitätsspektren zeigt, dass sich bei höheren Frequenzen der Einfluss der Absorption durch die Polymerfolie 66 auf den qualitativen Verlauf der Absorptionsintensität verringert. Durch Subtraktion oder Division der Absorptionsintensitätsspektren bei verschiedenen Pulsfrequenzen lassen sich so Einflüsse bestimmter Schichten weitgehend eliminieren bzw. Absorptionsintensitätsspektren bestimmter Stoffschichten errechnen, so dass bspw. ein Tiefenprofil hinsichtlich im Stoff 100 vorhandener Substanzen ermittelt werden kann.

Die Differenzbildung bzw. Division der Spektren kann beispielsweise, wie in Fig. 5 gezeigt, zu einem festgelegten Referenzspektrum erfolgen. Als Referenzspektrum kann beispielsweise das Spektrum bei der tiefsten oder der höchsten Pulsfrequenz dienen. Zur Berücksichtigung unterschiedlicher Pump-Intensitäten in den jeweiligen Schichten, hervorgerufen durch die Absorption höherer Schichten, kann der Subtraktion bzw. Division ein Bestimmen von Gewichtungsfaktoren vorausgehen, wobei die voneinander abzuziehenden oder durcheinander zu dividierenden Spektren bzw. Spektralwerte mit einem Gewichtungsfaktor zu multiplizieren sind. Zudem kann eine Hauptkomponenten Analyse nach dem Non-Linear Iterative Partial Least Squares (NIPALS) Algorithmus verwandt werden, beispielsweise zur Bestimmung einer Substanz, die sich in einer festen Matrix in der Tiefe unterschiedlich verteilt.

Ferner versteht es sich für den Fachmann, dass die oben gezeigten Ausführungsbeispiele rein illustrativ zu verstehen sind und den Schutzbereich der Ansprüche keinesfalls einschränken sollen. Insbesondere wird darauf hingewiesen, dass die spezifischen Anwendungen des Systems nicht auf die in den Figuren beschriebenen Anwendungen beschränkt sein sollen. Vielmehr wird davon ausgegangen, dass der Fachmann unmittelbar erkennt, dass die in den Figuren beschriebenen Anwendungen lediglich das erfinderische Prinzip verdeutlichen sollen, welches auf eine Vielzahl unterschiedlicher Stoffe und in den Stoffen enthaltene Substanzen anwendbar ist.

### BEZUGSZEICHENLISTE

- 10: Optisches Medium
- 11: Erste Oberfläche des optischen Mediums
- 12: Zweite Oberfläche des optischen Mediums
- 13: Dritte Oberfläche des optischen Mediums
- 14: Vierte Oberfläche des optischen Mediums
- 20: Quantenkaskadenlaser
- 21: Erster Infrarot-Laserstrahl-Teilweg
- 22: Optischer Chopper
- 23: Parabolspiegel
- 24: Zweiter Infrarot-Laserstrahl-Teilweg
- 25: Dritter Infrarot-Laserstrahl-Teilweg
- 30: Messlaser
- 31: Spiegel
- 40: Photodiode
- 41: Irisblende
- 50: Lock-In-Verstärker
- 51: Analog-zu-Digital-Wandler
- 52: Rechner
- 60: Justage-Laser
- 61: Spiegel
- 62: Spiegel
- 64: Glukoselösung
- 66: Polymerfolie

## Patentansprüche

1. Verfahren zum Analysieren eines Stoffes (100), umfassend die Schritte:
- Anordnen einer Stoffoberfläche des zu analysierenden Stoffes (100) auf einer Oberfläche (12) eines optischen Mediums (10), so dass zumindest ein Bereich der genannten Oberfläche (12) des optischen Mediums (10) in Kontakt mit der Stoffoberfläche steht;
- Aussenden eines Anregungs-Lichtstrahls mit einer Anregungswellenlänge durch den Bereich der Oberfläche (12) des optischen Mediums (10), der in Kontakt mit der Stoffoberfläche steht, auf die Stoffoberfläche, um eine Absorption von Anregungslicht in dem Stoff (100) zu gestatten, die eine Temperaturerhöhung induziert, die zu einer lokalen Änderung der Brechzahl bzw. des Brechungsindex des mit dem Stoff (100) in Kontakt stehenden optischen Mediums (10) führt;
- Aussenden eines Mess-Lichtstrahls durch das optische Medium (10) auf den Bereich der Oberfläche (12) des optischen Mediums (10), der in direktem Kontakt mit der Stoffoberfläche steht, so dass der Mess-Lichtstrahl an einer Grenzfläche des optischen Mediums (10) und der Stoffoberfläche reflektiert wird;
**dadurch gekennzeichnet, dass**
der Strahlengang des Mess-Lichtstrahls so ausgerichtet ist, dass er durch das optische Medium (10) auf den Bereich der Oberfläche ausgesendet wird, und sich die Auftrefforte des Mess-Lichtstrahls und des Anregungs-Lichtstrahls auf der Grenzfläche des optischen Mediums (10) und der Stoffoberfläche überlappen,
und dass das Verfahren die folgenden Schritte umfasst:
- Detektieren einer durch die genannte lokale Änderung der Brechzahl bzw. des Brechungsindex verursachten Ablenkung des reflektierten Mess-Lichtstrahls in Abhängigkeit von der Wellenlänge des Anregungs-Lichtstrahls; und
- Analysieren des Stoffes (100) anhand der detektierten Ablenkung des Mess-Lichtstrahls in Abhängigkeit von der Wellenlänge des Anregungs-Lichtstrahls, wobei der Grad der Ablenkung mit der Absorption des Anregungs-Lichtstrahls in dem Stoff korreliert.

2. Verfahren nach Anspruch 1, bei dem der Anregungs-Lichtstrahl ein Infrarot-Lichtstrahl ist.

3. Verfahren nach einem der vorgehenden Ansprüche, bei dem der Anregungs-Lichtstrahl ein intensitätsmodulierter Anregungs-Lichtstrahl ist, wobei die Modulationsfrequenz zwischen 5 und 2000 Hz liegt, und wobei der Schritt des Aussendens des Anregungs-Lichtstrahls vorzugsweise für verschiedene Modulationsfrequenzen wiederholt wird und der Schritt des Analysierens des Stoffes (100) das Analysieren des Stoffes (100) anhand der detektierten Ablenkungen des Mess-Lichtstrahls in Abhängigkeit von der Wellenlänge und der Modulationsfrequenz des Anregungs-Lichtstrahls umfasst.

4. Verfahren nach einem der vorhergehenden Ansprüche, bei dem die Wellenlänge des Anregungs-Lichtstrahls variiert wird, insbesondere
- die Wellenlänge zyklisch innerhalb eines vorbestimmten Wellenlängenbereiches durchgestimmt wird, oder
- charakteristische Wellenlängen, insbesondere Absorptions-Wellenlängen einer vermuteten Substanz, gezielt eingestellt werden.

5. Verfahren nach einem der vorhergehenden Ansprüche, bei dem der Anregungs-Lichtstrahl mit Hilfe eines Quantenkaskadenlasers (20) erzeugt wird, und/oder bei dem die Anregungs-Wellenlänge aus einem Bereich von 6 µm bis 13 µm, vorzugsweise von 8 µm bis 11 µm gewählt ist.

6. Verfahren nach einem der vorhergehenden Ansprüche, bei dem die Ablenkung des Mess-Lichtstrahls mit Hilfe eines positionsempfindlichen Photodetektors (PSD) ermittelt wird.

7. Verfahren nach einem der vorhergehenden Ansprüche, wobei der Stoff (100) die Haut eines Patienten ist und das Analysieren des Stoffes (100);
ein Bestimmen des Blutzuckerspiegels des Patienten,
ein Bestimmen eines Wassergehalts der Haut des Patienten,
ein Bestimmen einer Proteinzusammensetzung der Haut des Patienten, oder
ein Bestimmen einer Proteinzusammensetzung der Haut des Patienten in verschiedenen Hautschichten umfasst.

8. Verfahren nach Anspruch 7, bei dem der Wellenlänge des Anregungs-Lichtstrahls, basierend auf der detektierten Ablenkung des Mess-Lichtstrahls, ein Absorptionsintensitätswert zugeordnet wird, wobei das Analysieren des Stoffes (100) ein Bestimmen des Blutzuckerspiegels des Patienten umfasst und bei dem der Absorptionsintensitätswert mit einem Kalibrier-Absorptionsintensitätswert verglichen wird, welcher den Absorptionsintensitätswert der Haut eines Patienten bei einem bekanntem Blutzuckerspiegel und ebendieser Wellenlänge des Anregungs-Lichtstrahls repräsentiert, wobei der aktuelle Blutzuckerspiegel des Patienten vorzugsweise auf Basis des Vergleiches bestimmt wird, wobei der bestimmte Blutzuckerspiegel umso mehr von dem Blutzuckerspiegel während der Kalibrierung abweicht, je mehr der Absorptionsintensitätswert von dem Kalibrier-Absorptionsintensitätswert abweicht.

9. System zum Analysieren eines Stoffes (100), umfassend:
ein optisches Medium (10), welches eine Oberfläche (12) aufweist;
eine Einrichtung (20) zum Aussenden eines Anregungs-Lichtstrahls mit einer Anregungswellenlänge,
wobei die Einrichtung (20) zum Aussenden des Anregungs-Lichtstrahls so angeordnet ist, dass der ausgesendete Anregungs-Lichtstrahl in das optische Medium (10) eindringt und dieses an einem vorbestimmten Punkt an der Oberfläche (12) des optischen Mediums (10) wieder verlässt, wodurch gestattet wird, dass Anregungslicht in einen auf der Oberfläche (12) angeordneten zu analysierenden Stoff (100) eintritt, wenn eine Stoffoberfläche des zu analysierenden Stoffes (100) mit der genannten Oberfläche (12) des optischen Mediums (10) in Kontakt ist, um in dem zu analysierenden Stoff (100) absorbiert zu werden und
dadurch eine Temperaturerhöhung zu induzieren, die geeignet ist, zu einer lokalen Veränderung der Brechzahl bzw. des Brechungsindex des mit dem Stoff (100) in Kontakt stehenden optischen Mediums (10) zu führen; und
eine Messeinrichtung, wobei die Messeinrichtung eine Einrichtung (30) zum Aussenden eines Mess-Lichtstrahls umfasst, die so angeordnet ist, dass ein ausgesendeter Mess-Lichtstrahl in das optische Medium (10) eindringt und an einer Grenzfläche des optischen Mediums (10) und der Stoffoberfläche reflektiert wird, **dadurch gekennzeichnet, dass**
die Einrichtung (30) zum Aussenden der Mess-Lichtstrahls so angeordnet ist, dass im Betrieb der Strahlengang des Mess-Lichtstrahls so ausgerichtet ist, dass sich die Auftreffpunkte des Mess-Lichtstrahls und des Anregungs-Lichtstrahls auf der Grenzfläche des optischen Mediums (10) und der Stoffoberfläche überlappen, wobei die Messeinrichtung eine Einrichtung (40, 50, 51, 52) zum Empfangen des reflektierten Mess-Lichtstrahls und zum Detektieren einer Ablenkung des reflektierten Mess-Lichtstrahls umfasst, um zu gestatten, den Stoff (100) anhand der detektierten, durch die lokale Veränderung der Brechzahl bzw. des Brechungsindex verursachten Ablenkung des Mess-Lichtstrahls in Abhängigkeit von der Wellenlänge des Anregungs-Lichtstrahls zu analysieren, wobei der Grad der Ablenkung mit der Absorption des Anregungs-Lichtstrahls in dem Stoff korreliert.

10. System nach Anspruch 9, wobei der Mess-Lichtstrahl ein intensitätsmodulierter, insbesondere gepulster Mess-Lichtstrahl ist, wobei die Einrichtung (40, 41, 50, 51, 52) zum Empfangen des reflektierten Mess-Lichtstrahls und zum direkten oder indirekten Detektieren einer Ablenkung des reflektierten Mess-Lichtstrahls vorzugsweise einen Lock-in-Verstärker (50) umfasst, wobei vorzugsweise die Modulationsfrequenz, insbesondere die Pulsrate, zwischen 5 und 2000 Hz, vorzugsweise zwischen 10 und 1000 Hz und besonders vorzugsweise zwischen 20 und 700 Hz liegt.

11. System nach einem der Ansprüche 9 oder 10, wobei der Anregungs-Lichtstrahl ein Anregungs-Laserstrahl ist und die Einrichtung (20) zum Aussenden des Anregungs-Lichtstrahls dazu eingerichtet ist, Anregungs-Laserstrahlen unterschiedlicher Anregungs-Frequenzen auszusenden, wobei das System vorzugsweise eine optische Einrichtung umfasst, die geeignet ist, den Anregungs-Lichtstrahl auf den vorbestimmten Punkt zu fokussieren, sowie einen Justage-Laser (60) umfasst, welcher der Ausrichtung der optischen Einrichtung dient.

12. System nach einem der Ansprüche 9 bis 11, wobei die Einrichtung (40, 41, 50, 51, 52) zum Empfangen des reflektierten Mess-Lichtstrahls und zum direkten oder indirekten Detektieren einer Ablenkung des reflektierten Mess-Lichtstrahls
- einen Photodetektor, insbesondere eine Photodiode (40), und eine Irisblende (41) umfasst, wobei der Photodetektor hinter der Irisblende (41) angeordnet ist, oder
- einen positionsempfindlichen Photodetektor (PSD) umfasst, und/oder bei dem der Mess-Lichtstrahl vor der Detektion der Ablenkung mindestens ein weiteres Mal, vorzugsweise zwei bis fünf weitere Male in einen Überlappungsbereich mit dem Anregungs-Lichtstrahl auf der Grenzfläche zurückreflektiert wird.

13. Gerät zur Bestimmung des Blutzuckerspiegels eines Patienten, das folgendes umfasst:
- ein System nach einem der Ansprüche 9 bis 12,
- eine Steuerung zum Einstellen unterschiedlicher Wellenlängen des Anregungs-Lichtstrahls, und
- eine Logik- oder Recheneinheit (52), die dazu eingerichtet ist, aus detektierten Ablenkungen des Mess-Lichtstrahls in Abhängigkeit von der Anregungswellenlänge den Blutzuckerspiegel in der Haut eines Patienten zu bestimmen, wenn das optische Medium mit der Haut des Patienten derart in Kontakt gebracht wird, dass der an dem genannten vorbestimmten Punkt aus dem optischen Medium (10) austretende Anregungs-Lichtstrahl in die Haut eindringt.

14. Gerät zur Analyse eines Stoffes (100), das folgendes umfasst:
- ein System nach Anspruch 10,
- eine Steuerung zum Einstellen unterschiedlicher Modulationsfrequenzen des Anregungs-Lichtstrahls, und
- eine Logik- oder Recheneinheit (52), die dazu eingerichtet ist, den Stoff (100) vorzugsweise mittels detektierter Ablenkungen des Mess-Lichtstrahls bei unterschiedlichen Modulationsfrequenzen zu analysieren, wenn das optische Medium mit dem Stoff (100) derart in Kontakt gebracht wird, dass der an dem genannten vorbestimmten Punkt aus dem optischen Medium (10) austretende Anregungs-Lichtstrahl in den Stoff (100) eindringt, wobei der Stoff (100) vorzugsweise die Haut eines Patienten ist, und die Logik- oder Recheneinheit (52) dazu eingerichtet ist, aus detektierten Ablenkungen des Mess-Lichtstrahls bei unterschiedlichen Modulationsfrequenzen, unterschiedliche Schichten der Haut des Patienten zu analysieren.

15. Gerät zur Analyse von Inhaltsstoffen einer Flüssigkeit oder Emulsion, das folgendes umfasst:
- ein System nach einem der Ansprüche 9 bis 12,
- eine Steuerung zum Einstellen unterschiedlicher Wellenlängen des Anregungs-Lichtstrahls, und
- eine Logik- oder Recheneinheit (52), die dazu eingerichtet ist, aus detektierten Ablenkungen des Mess-Lichtstrahls in Abhängigkeit von der Anregungswellenlänge Inhaltsstoffe der Flüssigkeit oder Emulsion zu bestimmen, wenn das optische Medium mit der Flüssigkeit oder Emulsion derart in Kontakt gebracht wird, dass der an dem genannten vorbestimmten Punkt aus dem optischen Medium (10) austretende Anregungs-Lichtstrahl in die Flüssigkeit oder Emulsion eindringt.

## Claims

1. A method for analyzing a substance (100), comprising the following steps:
- arranging a surface of said substance (100) to be analyzed on a surface (12) of an optical medium (10), so that at least a portion of the surface (12) of the optical medium (10) is in contact with the substance surface;
- emitting an excitation light beam with an excitation wavelength through the region of the surface (12) of the optical medium (10) in contact with the substance surface, onto the substance surface, to enable an absorption of excitation light in the substance (100) inducing a temperature increase, which leads to a local change in the refraction number or refraction index of the optical medium (10) in contact with said substance (100);
- emitting a measurement light beam through the optical medium (10) onto the region of the surface (12) of the optical medium (10) which is in direct contact with the substance surface, such that the measurement light beam is reflected at an interface of the optical medium (10) and the substance surface;
**characterized in that** the light path of the measurement light beam is arranged such that it is emitted through the optical medium (10) to said region of the surface (12), and that the locations where the measurement light beam and the excitation light beam impinge onto the interface of the optical medium (10) and the surface of the substance overlap, and that the method comprises the following steps:
- detecting a deflection of the reflected measurement light beam, which is caused by said local change in the refraction number or refraction index, as dependent on the wavelength of the excitation light beam; and
- analyzing the substance (100) on the basis of the detected deflection of the measurement light beam as dependent on the wavelength of the excitation light beam, wherein the extent of the deflection correlates with the absorption of the excitation light beam in said substance.

2. The method according to Claim 1, in which the excitation light beam is an infrared light beam.

3. The method according to any one of the previous claims, in which the excitation light beam is an intensity-modulatedexcitation light beam, wherein
the modulation frequency is between 5 and 2000 Hz, and
wherein the step of emitting the excitation light beam preferably is repeated for different modulation frequencies and the step of analyzing the substance (100) comprises the analysis of the substance (100) on the basis of the detected deflections of the measurement beam as dependent on the wavelength and the modulation frequency of the excitation light beam.

4. The method according to any of the preceding claims, in which the wavelength of the excitation light beam is varied, wherein in particular
- the wavelength is cyclically tuned within a predetermined wavelength range, or
- characteristic wavelengths, in particular absorption wavelengths of a suspected substance, are selectively set.

5. The method according to any one of the preceding claims, in which the excitation light beam is generated by means of a quantum cascade laser (20), and/or
in which the excitation wavelength is selected from a range of 6 µm to 13 µm, preferably of 8 µm to 11 µm.

6. The method according to any one of the preceding claims, in which the deflection of the measurement light beam is determined by means of a position-sensitive photo-detector (PSD).

7. The method according to any one of the preceding claims, wherein the substance (100) is the skin of a patient and the analysis of the substance (100) comprises:
determining a blood sugar level of the patient,
determining a water content of the skin of the patient,
determining a protein composition of the skin of the patient, or
determining a protein composition of the skin of the patient in different skin layers.

8. The method according to claim 7, in which, based on the detected deflection of the measurement light beam an absorption intensity value is associated with the wavelength of the excitation light beam,
wherein the analysis of the substance (100) comprises determining the blood sugar level of the patient and wherein the absorption intensity value is compared with a calibration absorption intensity value, which represents the absorption intensity value of the skin of a patient at a known blood sugar level and at this exact wavelength of the excitation light beam,
wherein the current blood sugar level of the patient preferably is determined on the basis of the comparison, wherein the determined blood sugar level deviates the more from the blood sugar level during calibration, the more the absorption intensity value deviates from the calibration absorption intensity value.

9. A system for analyzing a substance (100), comprising:
an optical medium (10) having a surface (12);
a device (20) for emitting an excitation light beam with an excitation wavelength, wherein the device (20) for emitting the excitation light beam is arranged such that the emitted excitation light beam enters the optical medium (10) and exits the same again at a predetermined point on the surface (12) of the optical medium (10), thereby enabling excitation light to enter a substance (100) to be analysed, which is arranged on the surface (12), when the surface of the substance (100) to be analysed is in contact with said surface (12) of the optical medium (10), to be absorbed in the substance (100) to be analysed and to thereby induce in increase in temperature, which is suitable to cause a local change in the refraction number or refraction index of the optical medium (10) which is in contact with said substance (100); and
a measurement device, wherein the measurement device comprises a device (30) for emitting a measurement light beam, which is arranged such that an emitted measurement light beam enters the optical medium (10), and is reflected an interface of the optical medium (10) and the substance surface, **characterized in that** said device (30) for emitting said measurement light beam is arranged such that during operation the light path of said measurement light beam is arranged such that the locations where the measurement light beam and the excitation light beam impinge on the interface of the optical medium (10) and the substrate surface overlap,
and wherein the measurement device comprises a device (40, 50, 51, 52) for receiving the reflected measurement light beam and for detecting a deflection of the reflected measurement light beam, to allow for analysing the substance (100) based on the deflection of the measurement light beam, caused by the local change of the refraction number or refraction index, as dependent on the wavelength of the excitation light beam, wherein the extent of the deflection correlates with the absorption of the excitation-light beam.

10. The system according to claim 9, wherein the measurement light beam is an intensity-modulated, in particular pulsed, measurement light beam,
wherein the device (40, 41, 50, 51, 52) for receiving the reflected measurement light beam and for directly or indirectly detecting a deflection of the reflected measurement light beam preferably comprises a lock-in amplifier (50),
wherein the modulation frequency, in particular the pulse rate, preferably is between 5 and 2000 Hz, preferably between 10 and 1000 Hz, and particularly preferably between 20 and 700 Hz.

11. The system according to any one of Claims 9 or 10, wherein the excitation light beam is an excitation laser beam and the device (20) for emitting the excitation light beam is configured for emitting excitation laser beams of different excitation frequencies wherein the system preferably comprises
an optical device which is suitable for focussing the excitation light beam on the predetermined location and an alignment laser (60) for aligning the optical device.

12. The system according to any one of Claims 9 to 11, wherein the device (40, 41, 50, 51, 52) for receiving the reflected measurement light beam and for directly or indirectly detecting a deflection of the reflected measurement light beam
- comprises a photo-detector, in particular a photodiode (40), and an iris diaphragm (41), wherein the photo-detector is arranged behind the iris diaphragm (41), or
- comprises a position-sensitive photo-detector (PSD), and/or
in which before the detection of the deflection, the measurement light beam is reflected back into a region of overlap with the excitation light beam on the interface at least one additional time, preferably two to five additional times.

13. The apparatus for determining the blood sugar level of a patient, which comprises the following:
- a system according to any one of Claims 9 to 12,
- a controller for setting different wavelengths of the excitation light beam, and
- a logic unit or calculating unit (52), which is configured to determine blood sugar levels in the skin of a patient from detected deflections of the measurement light beam as a function of the excitation wavelength, when the optical medium is brought into contact with the skin of the patient in such a way that the excitation light beam emerging from the optical medium (10) at the said predetermined point enters into the skin.

14. An apparatus for analyzing a substance (100), which comprises the following:
- a system according to Claim 10,
- a controller for setting different modulation frequencies of the excitation light beam, and
- a logic unit or calculating unit (52), which is configured to analyze the substance (100) preferably by means of detected deflections of the measurement light beam at different modulation frequencies, when the optical medium is brought into contact with the substance (100) in such a way that the excitation light beam emerging from the optical medium (10) at the said predetermined point enters into the substance (100),
wherein the substance (100) preferably is the skin of a patient, and the logic unit or calculating unit (52) is configured to analyze different layers of the skin of the patient from detected deflections of the measurement light beam at different modulation frequencies.

15. An apparatus for analyzing constituents of a liquid or emulsion, comprising the following:
- a system according to any one of Claims 9 to 12,
- a controller for setting different wavelengths of the excitation light beam, and
- a logic unit or calculating unit (52), which is configured to determine constituents of the liquid or emulsion from detected deflections of the measurement light beam as a function of the excitation wavelength, when the optical medium is brought into contact with the liquid or emulsion in such a way that the excitation light beam emerging from the optical medium (10) at the said predetermined point enters into the liquid or emulsion.

## Revendications

1. Procédé pour l'analyse d'une substance (100), comprenant les étapes suivantes :
- mise en place d'une surface de la substance à analyser (100) sur une surface (12) d'un milieu optique (10) de telle sorte qu'au moins une zone de la surface mentionnée (12) du milieu optique (10) soit en contact avec la surface de la substance ;
- émission d'un faisceau lumineux d'excitation, présentant une longueur d'onde d'excitation, à travers la zone de la surface (12) du milieu optique (10) qui est en contact avec la surface de la substance, sur la surface de la substance, pour permettre une absorption de la lumière d'excitation par la substance (100), qui induit une élévation de la température qui conduit à une variation de la valeur de la réfraction ou de l'indice de réfraction du milieu optique (10) en contact avec la substance (100) ;
- émission d'un faisceau lumineux de mesure à travers le milieu optique (10) sur la zone de la surface (12) du milieu optique (10) qui est en contact direct avec la surface de la substance, de telle sorte que le faisceau lumineux de mesure soit réfléchi sur une surface limite du milieu optique (10) et de la surface de la substance ;
**caractérisé en ce que** la trajectoire du faisceau lumineux de mesure est dirigée de telle sorte qu'il soit émis, par le milieu optique (10), sur la zone de la surface, et que les points d'incidence du faisceau lumineux de mesure et du faisceau lumineux d'excitation sur la surface limite du milieu optique (10) et de la surface de la substance se chevauchent,
et **en ce que** le procédé comprend les étapes suivantes :
- détection d'une déviation du faisceau lumineux de mesure réfléchi, provoquée par la variation locale mentionnée de la valeur de la réfraction ou de l'indice de réfraction, en fonction de la longueur d'onde du faisceau lumineux d'excitation ; et
- analyse de la substance (100) à l'aide de la déviation détectée du faisceau lumineux de mesure en fonction de la longueur d'onde du faisceau lumineux d'excitation, le degré de déviation présentant une corrélation avec l'absorption du faisceau lumineux d'excitation par la substance.

2. Procédé selon la revendication 1, dans lequel le faisceau lumineux d'excitation est un faisceau lumineux infrarouge.

3. Procédé selon l'une des revendications précédentes, dans lequel le faisceau lumineux d'excitation est un faisceau lumineux d'excitation modulé en intensité, la fréquence de modulation étant comprise entre 5 et 2000 Hz, et l'étape d'émission du faisceau lumineux d'excitation étant de préférence répétée pour différentes fréquences de modulation, et l'étape d'analyse de la substance (100) comprenant l'analyse de la substance (100) à l'aide des déviations détectées du faisceau lumineux de mesure en fonction de la longueur d'onde et de la fréquence de modulation du faisceau lumineux d'excitation.

4. Procédé selon l'une des revendications précédentes, dans lequel on fait varier la longueur d'onde du faisceau lumineux d'excitation, en particulier
- la longueur d'onde est ajustée d'une manière cyclique à l'intérieur d'une plage prédéfinie de longueurs d'onde, ou
- des longueurs d'onde caractéristiques sont soumises à un réglage ciblé, en particulier les longueurs d'onde d'absorption d'une substance suspectée.

5. Procédé selon l'une des revendications précédentes, dans lequel le faisceau lumineux d'excitation est produit à l'aide d'un laser à cascade quantique (20), et/ou dans lequel la longueur d'onde d'excitation est choisie dans une plage de 6 µm à 13 µm, de préférence de 8 µm à 11 µm.

6. Procédé selon l'une des revendications précédentes, dans lequel la déviation du faisceau lumineux de mesure est déterminée à l'aide d'un photodétecteur détecteur de position (PSD).

7. Procédé selon l'une des revendications précédentes, dans lequel la substance (100) est la peau d'un patient, et l'analyse de la substance (100) comprend
une détermination de la glycémie du patient,
une détermination de la teneur en eau de la peau du patient,
une détermination de la composition en protéines de la peau du patient, ou
une détermination de la composition en protéines de la peau du patient dans différentes couches cutanées.

8. Procédé selon la revendication 7, dans lequel une valeur de l'intensité d'absorption est affectée à la longueur d'onde du faisceau lumineux d'excitation, sur la base de la déviation détectée du faisceau lumineux de mesure, l'analyse de la substance (100) comprenant une détermination de la glycémie du patient, et la valeur de l'intensité d'absorption étant comparée à une valeur d'étalonnage de l'intensité d'absorption, qui représente la valeur de l'intensité d'absorption de la peau d'un patient en présence d'une glycémie connue et pour cette même longueur d'onde du faisceau lumineux d'excitation, la glycémie actuelle du patient étant déterminée de préférence sur la base de la comparaison, la glycémie déterminée s'écartant d'autant plus de la glycémie pendant l'étalonnage que la valeur de l'intensité d'absorption s'écarte plus de la valeur d'étalonnage de l'intensité d'absorption.

9. Système pour l'analyse d'une substance (100), comprenant :
un milieu optique (10), qui présente une surface (12) ;
un dispositif 20 pour l'émission du faisceau lumineux d'excitation à une longueur d'onde d'excitation,
le dispositif (20) pour l'émission du faisceau lumineux d'excitation étant agencé de telle sorte que le faisceau lumineux d'excitation émis pénètre dans le milieu optique (10) et quitte de nouveau ce dernier en un point prédéfinie sur la surface (12) du milieu optique (10), ce qui permet à la lumière d'excitation de pénétrer dans une substance à analyser (100), disposée sur la surface (12), quand une surface de la substance (100) à analyser est en contact avec la surface mentionnée (12) du milieu optique (10), pour être absorbée par la substance à analyser (100), et induisant de ce fait une élévation de la température, qui est apte à conduire à une variation locale de la valeur de la réfraction ou de l'indice de réfraction du milieu optique (10) en contact avec la substance (100) ; et
un dispositif de mesure, le dispositif de mesure comprenant un dispositif (30) pour l'émission d'un faisceau lumineux de mesure, qui est agencé de façon qu'un faisceau lumineux de mesure émis pénètre dans le milieu optique (10) et soit réfléchi par une surface limite du milieu optique (10) et de la surface de la substance, **caractérisé en ce que**
le dispositif (30) pour l'émission du faisceau lumineux de mesure est agencé de telle sorte que, en cours d'utilisation, la trajectoire du faisceau lumineux de mesure soit orientée de telle sorte que les points d'incidence du faisceau lumineux de mesure et du faisceau lumineux d'excitation sur la surface limite du milieu optique (10) et de la surface de la substance se chevauchent, le dispositif de mesure comprenant un dispositif (40, 50, 51, 52) pour la réception du faisceau lumineux de mesure réfléchi et pour la détection d'une déviation du faisceau lumineux de mesure réfléchie, pour permettre une analyse de la substance (100) à l'aide de la déviation du faisceau lumineux de mesure telle que détectée, provoquée par la variation locale de la valeur de la réfraction ou de l'indice de réfraction, en fonction de la longueur d'onde du faisceau lumineux d'excitation, le degré de déviation présentant une corrélation avec l'absorption du faisceau lumineux d'excitation par la substance.

10. Système selon la revendication 9, dans lequel le faisceau lumineux de mesure est un faisceau lumineux de mesure à modulation d'intensité, en particulier pulsé, le dispositif (40, 41, 50, 51, 52) pour l'émission du faisceau lumineux de mesure réfléchi et pour la détection directe ou indirecte d'une déviation du faisceau de mesure réfléchi comprenant de préférence un amplificateur de blocage (50), la fréquence de modulation, en particulier le taux d'impulsion, étant de préférence comprise entre 5 et 2000 Hz, de préférence entre 10 et 1000 Hz et d'une manière particulièrement préférée entre 20 et 700 Hz.

11. Système selon l'une des revendications 9 ou 10, dans lequel le faisceau lumineux d'excitation est un faisceau laser d'excitation et le dispositif (20) pour l'émission du faisceau lumineux d'excitation est agencé de façon à émettre des faisceaux lumineux d'excitation à différentes fréquences d'excitation, le système comprenant de préférence un dispositif optique apte à focaliser le faisceau lumineux d'excitation sur le point prédéfini, ainsi qu'un laser d'alignement (60), qui sert à l'orientation du dispositif optique.

12. Système selon l'une des revendications 9 à 11, dans lequel le dispositif (40, 41, 50, 51, 52) pour l'émission du faisceau lumineux de mesure réfléchi et pour la détection directe ou indirecte d'une déviation du faisceau lumineux de mesure réfléchi
- comprend un photodétecteur, en particulier une photodiode (40) et un diaphragme à iris (41), le photodétecteur étant disposé derrière le diaphragme à iris (41), ou
- comprend un photodétecteur détecteur de position (PSD), et/ou dans lequel le faisceau lumineux de mesure subit, avant la détection de la déviation, une nouvelle réflexion au moins une fois supplémentaire, de préférence encore deux à cinq fois, dans une zone de chevauchement avec le faisceau lumineux d'excitation sur la surface limite.

13. Appareil pour la détermination de la glycémie d'un patient, qui comprend ce qui suit :
- un système selon l'une des revendications 9 à 12,
- une commande pour le réglage de différentes longueurs d'onde du faisceau lumineux d'excitation, et
- une unité logique ou de calcul (52), qui est agencée pour déterminer à partir de déviations détectées du faisceau lumineux de mesure, en fonction de la longueur d'onde d'excitation, la glycémie dans la peau d'un patient, quand le milieu optique est mis en contact avec la peau du patient de telle sorte que le faisceau lumineux d'excitation sortant du milieu optique au niveau du point prédéfini mentionné pénètre dans la peau.

14. Appareil pour l'analyse d'une substance (100), qui comprend :
- un système selon la revendication 10,
- une commande pour le réglage de différentes fréquences de modulation du faisceau lumineux d'excitation, et
- une unité logique ou de calcul (52), qui est agencée pour analyser la substance (100) de préférence à l'aide de déviations détectées du faisceau lumineux de mesure en présence de différentes fréquences de modulation, quand le milieu optique est mis en contact avec la substance (100) de telle sorte que le faisceau lumineux d'excitation sortant du milieu optique (10) au niveau du point prédéfini mentionné pénètre dans la substance (100), la substance (100) étant de préférence la peau d'un patient, et l'unité logique ou de calcul (52) étant agencée de façon à analyser, à partir de déviations détectées du faisceau lumineux de mesure, pour différentes fréquences de modulation, différentes couches de la peau du patient.

15. Appareil pour l'analyse des constituants d'un liquide ou d'une émulsion, qui comprend ce qui suit :
- un système selon l'une des revendications 9 à 12,
- une commande pour le réglage de différentes longueurs d'onde du faisceau lumineux d'excitation, et
- une unité logique ou de calcul (52), qui est agencée pour déterminer, à partir de déviations détectées du faisceau lumineux de mesure, en fonction de la longueur d'onde d'excitation, les constituants du liquide ou de l'émulsion, quand le milieu optique est mis en contact avec le liquide ou l'émulsion de telle sorte que le faisceau lumineux d'excitation sortant du milieu optique (10) au niveau du point prédéfini mentionné pénètre dans le liquide ou l'émulsion.
